Europäisches Patentamt

European Patent Office

Office européen des brevets

(19)

(11) Numéro de publication : **0 521 758 A1**

(12) **DEMANDE DE BREVET EUROPEEN**

(21) Numéro de dépôt : **92401826.0**

(22) Date de dépôt : **26.06.92**

(51) Int. Cl.⁵ : **C12N 1/20,** C12Q 1/24,
A01N 63/00

Le (Les) microorganisme(s) a (ont) été déposé(s) auprès * sous le(s) numéro(s) I-1112, I-1113, I-1114, and I-1115

(30) Priorité : **26.06.91 FR 9107882**

(43) Date de publication de la demande :
**07.01.93 Bulletin 93/01**

(84) Etats contractants désignés :
**PT**

(71) Demandeur : **INSTITUT NATIONAL DE LA RECHERCHE AGRONOMIQUE (INRA)**
**147, Rue de l'Université**
**F-75007 Paris Cédex 07 (FR)**

(72) Inventeur : **Garbaye, Jean**
**4, rue Beauveau**
**F-54000 Nancy (FR)**
Inventeur : **Duponnois, Robin**
**Rue saint Aignan**
**F-52000 Chaumont (FR)**

(74) Mandataire : **Peaucelle, Chantal**
**Cabinet Armengaud Aine 3, avenue Bugeaud**
**F-75116 Paris (FR)**

(54) **Moyens pour améliorer la croissance des plantes.**

(57) L'invention concerne des moyens pour améliorer la croissance de plantes constitués par des souches bactériennes capables de stimuler spécifiquement la formation de mycorhizes par des champignons mycorhiziens inoculés, ces souches exerçant un effet antagoniste vis-à-vis de champignons contaminants compétiteurs vis-à-vis du champignon inoculé.

EP 0 521 758 A1

L'invention a pour objet des moyens pour améliorer la croissance des plantes, plus spécialement des arbres forestiers, en perfectionnant les techniques de mycorhization contrôlée.

On sait que bon nombre de plantes, et notamment les arbres forestiers, dès le stade semis, vivent en symbiose obligatoire avec des champignons qui leur sont associés au niveau des racines. Les organes mixtes racine et champignon portent le nom de mycorhizes. L'efficacité de la symbiose, appréciée en terme de stimulation de la croissance d'une plante donnée, dans des conditions environnementales déterminées, varie fortement selon l'identité du champignon associé. Pour une culture donnée, les champignons symbiotiques potentiels sont en très grand nombre. On appelle "mycorhization contrôlée" l'ensemble des techniques qui visent à orienter artificiellement la mycorhization dans un sens favorable à la croissance des plantes, par exemple en inoculant les plantes avec des souches fongiques sélectionnées pour leur efficacité supérieure à la moyenne.

Pour améliorer l'effet des inoculums fongiques, il est possible de jouer sur des facteurs culturaux tels que la fertilisation ou la désinfection du sol. Ce dernier aspect est particulièrement important. En effet, le succès de l'inoculation mycorhizienne ne peut être assuré qu'en l'absence de compétition de la part d'autres champignons symbiotiques. On mesurera donc que toute innovation réduisant cette compétition et les besoins de désinfection serait un progrès en matière de mycorhization contrôlée.

Les co-inventeurs ont également décrit l'obtention de bactéries capables de stimuler la mycorhization d'essences forestières telles que le Douglas. Ces co-inventeurs ont ainsi défini le concept de bactéries auxiliaires de la mycorhization (BAM).

La poursuite des travaux dans ce domaine a amené les inventeurs à mettre en évidence que certaines bactéries, étroitement associées à des champignons mycorhiziens en symbiose avec une plante hôte, constituent des auxiliaires spécifiques de la mycorhization de grande valeur, favorisant ainsi indirectement la croissance de ces plantes.

L'invention a donc pour but de fournir de nouvelles bactéries capables d'accélérer fortement la symbiose entre un champignon mycorhizien et la plante hôte, et par là de permettre une augmentation de la croissance de la plante.

Elle a également pour but de fournir un procédé d'isolement de ces souches.

L'invention a également pour but de fournir un procédé de mycorhization de grande efficacité et des compositions utilisables dans la mise en oeuvre de ce procédé.

Les souches bactériennes de l'invention sont caractérisées en ce qu'elles sont capables de stimuler spécifiquement la formation de mycorhizes par des champignons mycorhiziens, ces souches exerçant un effet antagoniste vis-à-vis de champignons compétiteurs du champignon inoculé.

L'effet stimulant spécifique vis-à-vis du champignon inoculé est tel que le taux de mycorhization, c'est-à-dire le rapport entre le pourcentage de racines mycorhizées formées et le nombre total de racines, est significativement accru d'au moins 10 % environ par rapport au système sans apport de bactéries.

Ceci étant, l'homme de l'art appréciera aisément au regard d'un système plante, champignon, culture, le seuil à partir duquel le taux de mycorhization se traduit par un avantage pratique.

De la même façon, l'effet antagoniste vis-à-vis d'un champignon compétiteur est tel que le pourcentage de racines mycorhizées formées par celui-ci est significativement réduit à une valeur inférieure à 10 % environ.

On mesurera l'intérêt de ce double effet qui permet de valoriser le principe de la mycorhization contrôlée.

L'invention fournit en effet des moyens à la fois pour renforcer et accélérer l'établissement de la symbiose entre le champignon inoculé et la plante hôte et pour inhiber l'établissement du champignon compétiteur. Il est ainsi possible de s'affranchir de l'opération de désinfection du sol qui est actuellement indispensable avant semis et inoculation.

Des souches bactériennes selon l'invention sont constituées par des bactéries Gram positif du genre Bacillus.

Comme bactéries de ce genre, on citera par exemple, celles de l'espèce Bacillus subtilis , en particulier celle appelée ci-après A2 déposée à la C.N.C.M. (Collection Nationale de Cultures de Microorganismes) le 11 juin 1991 sous le n° I-1112 et Bacillus sp (ci-après désignée A15) déposée à la C.N.C.M. le même jour, sous le n° I-1113.

D'autres bactéries de grand intérêt pour la mycorhization sont des bactéries Gram négatif du genre Pseudomonas.

Parmi les bactéries de ce genre, celles de l'espèce Pseudomonas fluorescens exercent des effets agonistes et antagonistes, tels que définis ci-dessus, particulièrement avantageux.

Les souches Pseudomonas sp et Pseudomonas fuorescens (appelées ci-après respectivement B5 et B8), déposées à la C.N.C.M. le 11 juin 1991 sous les numéros respectifs I-1114 et I-1115, présentent à cet égard une grande efficacité. Ces souches ont fait l'objet d'une caractérisation moléculaire. Comme rapporté dans l'exemple 1, la souche B8 est caractérisée par la présence dans son ADN ribosomal 16S de la séquence :

5'-ATGTAAGGCCATGGTAAGTTCTTCGACGATTT-3'.

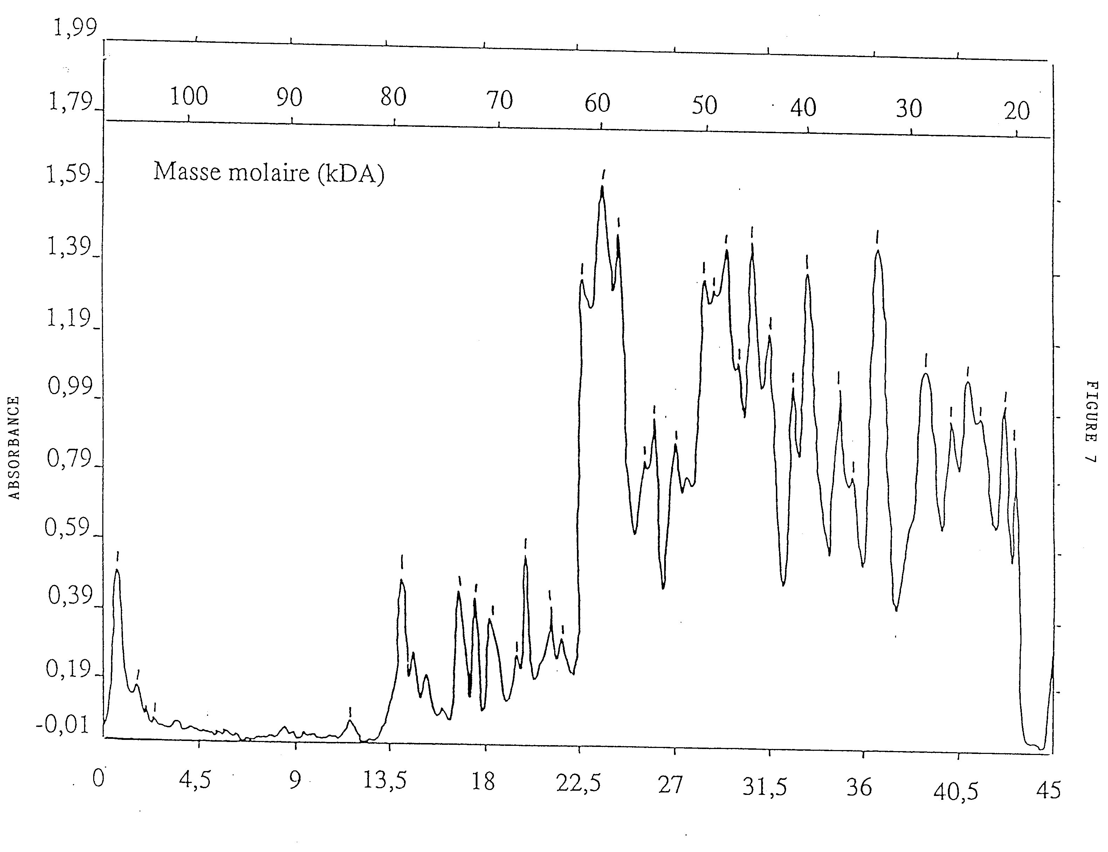

Cette souche est également caractérisée par son profil polypeptidique, tel que représenté sur la figure 7, obtenu par électrophorèse monodimensionnelle en conditions dénaturantes sur gel.

L'invention vise plus spécialement les souches bactériennes provenant de la rhizosphère des arbres forestiers.

Selon un autre aspect, les souches bactériennes de l'invention sont caractérisées en ce qu'elles sont telles qu'isolées des manteaux de mycorhizes et des carpophores des champignons ectomycorhiziens.

Les bactéries de l'invention constituent des auxiliaires de grand intérêt vis-à-vis de champignons ectomycorhiziens.

Les ectomycorhizes se rencontrent chez de nombreuses espèces forestières. Elles sont formées par de nombreux champignons basidiomycètes et ascomycètes supérieurs.

Des champignons de ce type ayant une efficacité symbiotique particulièrement élevée, comprennent Laccaria sp (par exemple : Laccaria laccata, Laccaria bicolor), Hebeloma sp (par exemple : H.crustuliniforme, H.cylindrosporus), Paxillus involutus, Rhizopogon sp, (par exemple R.vinicolor), Suillus sp, Scleroderma sp, Boletus edulis..

L'efficacité des souches de l'invention vis-à-vis de champignons compétiteurs des champignons ectomycorhiziens ci-dessus est particulièrement élevée.

L'effet antagoniste vis-à-vis par exemple de T.terrestris, l'un des contaminants les plus répandus en pépinière, conduit à une inhibition pratiquement totale du développement de ce champignon.

Cet effet est également important vis-à-vis de souches indésirables par rapport à un champignon sélectionné donné, qui peuvent donc comprendre l'un des champignons mentionnés plus haut, dès lors qu'il n'a pas été choisi pour la mycorhization.

La sélectivité bactérienne sur le champignon ectomycorhizien met en jeu l'émission de composés gazeux par les isolats bactériens.

Ainsi, l'analyse chromatographique en phase gazeuse couplée à la spectrométrie de masse, a permis d'identifier, chez des cultures de bactéries de type Pseudomonas, extraites à l'aide d'éther éthylique, divers composés gazeux, tels que l'acide oléique, l'alcool isoamylique et le phényl-2 éthanol. L'émission de tels composés constitue donc une caractéristique des souches de l'invention.

Les souches bactériennes de l'invention sont également caractérisées par leur capacité à dégrader le ciment intercellulaire et à faciliter la pénétration des hyphes entre les cellules grâce à au moins l'une des activités enzymatiques suivantes : endoglucanase, cellobiose hydrolase, pectate lysase ou xylanase.

Leur pouvoir de survie et de colonisation du sol est élevé comme le montre leur activité lipase, amylase ou protéase.

Selon encore un autre aspect, les souches bactériennes de l'invention sont caractérisées en ce qu'elles sont telles qu'obtenues par un procédé comprenant :
- la mise en suspension de carpophores et de mycorhizes prélevées sur Laccaria laccata en symbiose avec Douglas,
- la culture en milieu approprié, tel que TSA, selon les techniques habituelles,
- l'addition d'un inoculum de L.Laccata,
- l'ensemencement avec des graines de Douglas,
- la sélection in vitro, en serre ou en pépinière, des isolats bactériens capables de donner lieu à un taux de mycorhization d'au moins 80 % environ par L.laccata et d'inhiber pratiquement totalement la formation de mycorhizes par des champignons compétiteurs tels que T.terrestris.

L'invention vise également un procédé d'obtention de souches bactériennes telles que définies ci-dessus. Ce procédé est caractérisé en ce qu'il comprend :
- la mise en suspension de carpophores et de mycorhizes prélevés sur des champignons mycorhiziens associés à des plantes hôtes,
- la dilution en série de ces suspensions et leur application sur un milieu approprié pour leur croissance,
- la sélection des isolats capables de donner lieu à un taux de mycorhization, sur un champignon mycorhizien inoculé à une plante hôte, significativement accru d'au moins 10 % environ par rapport au système sans apport de bactéries et d'inhiber pratiquement totalement l'établissement de champignons contaminants, compétiteurs vis-à-vis du champignon de l'inoculum.

Les tests effectués dans des conditions variées, synthèse in vitro dans des conditions microbiennes contrôlées, essais en pots en serre, essais en pépinières, ont montré que les bactéries définies ci-dessus, renforcent et accélèrent fortement l'établissement de la symbiose entre une plante hôte et un champignon mycorhizien et donc la croissance de la plante et ce, tout en inhibant le développement de champignons compétiteurs inefficaces vis-à-vis de la symbiose. Elles constituent donc des adjuvants biologiques de grand intérêt pour les procédés de mycorhization.

L'effet de ces adjuvants est en outre durable puisqu'il s'exerce sur le champignon en symbiose avec la plante hôte, ce qui distingue totalement les bactéries de l'invention des PGPR classiques isolées du sol ou de la rhizosphère.

L'invention vise donc l'utilisation des souches bactériennes ci-dessus pour l'élaboration des compositions utilisables comme adjuvants pour la mycorhization contrôlée de plantes.

Ces compositions sont caractérisées en ce qu'elles comprennent une souche bactérienne telle que définie plus haut, en association avec un véhicule solide ou liquide, inerte.

Il s'agit notamment d'une suspension bactérienne renfermant avantageusement de $10^7$ à $10^{12}$, de préférence de $10^8$ à $10^{10}$ bactéries/l. Ces suspensions contiennent des adjuvants nécessaires à la conservation tel que le sulfate de magnésium, le polyéthylène glycol ou le glycérol. Ces doses seront aisément ajustées par l'homme de l'art en tenant compte de l'efficacité d'une souche donnée.

Le milieu de suspension est un véhicule liquide inerte. De manière pratique, on peut utiliser de l'eau stérile.

La suspension est diluée au moment de l'emploi.

Cette suspension peut être inoculée en mélange avec un véhicule solide, tel qu'un gel, ou un matériau particulaire poreux, inerte vis-à-vis des bactéries.

Des matériaux gélifiables appropriés comprennent des polymères d'origine végétale ou animale, les hydrogels, les alginates étant particulièrement préférés.

Parmi les matériaux particulaires, on citera la vermiculite, la tourbe, ou leurs mélanges, et l'argile expansée.

En variante, la souche bactérienne se présente sous forme d'un inoculum liquide ou solide mixte avec le champignon.

Dans cette forme de réalisation, la bactérie est incorporée dans l'inoculum mycorhizien, avantageusement à des doses de $10^6$ à $10^9$ bactéries par gramme de poids sec de mycélium. On utilise un véhicule permettant de manipuler et d'appliquer l'inoculum aisément. Il s'agit plus spécialement d'un véhicule solide, notamment d'un gel ou d'un matériau particulaire poreux tel qu'évoqué ci-dessus.

Un inoculum mixte avantageux se présente ainsi sous forme de billes d'alginate de calcium.

En variante, l'inoculum peut être directement associé à la graine.

Des inoculums mixtes particulièrement préférés pour la culture de résineux comme le Douglas, ou le chêne, comprennent au moins l'une des bactéries de l'invention en association avec L.laccata.

L'invention vise en particulier les inoculums mixtes de L.laccata avec l'une des bactéries déposées à la C.N.C.M., mentionnées plus haut.

Pour la culture du hêtre, des inoculums mixtes plus spécialement appropriés comprennent H.crustuliniforme ou P.involutus associés aux bactéries de l'invention.

D'une manière générale, l'inoculum mycélien utilisé conjointement ou non avec la bactérie est préparé avantageusement comme décrit dans "Les mycorhizes des arbres et des plantes cultivées", par D.G. Strullu, éd. Lavoisier, Paris, 1991.

Cette préparation comprend la fermentation solide du substrat.

En variante, on réalise une fermentation liquide suivie de l'inclusion dans un véhicule inerte tel qu'un gel.

L'invention vise également un procédé de mycorhization contrôlée de plantes caractérisé en ce qu'il comprend l'utilisation d'une quantité efficace de souche bactérienne telle que définie plus haut.

L'invention vise en particulier la mycorhization d'essences forestières.

Comme rapporté dans les exemples, il s'avère en outre que ces bactéries ne sont pas spécifiques d'une essence forestière donnée, ce qui leur confère un large champ d'application.

Parmi les essences les plus utilisées en reboisement et en régénération artificielle, qui sont particulièrement appropriées pour la mise en oeuvre de l'invention, on citera les résineux comme le Douglas, les épicéas, les pins, les mélèzes et les tsugas ou des espèces feuillues comme les chênes, les hêtres et les eucalyptus.

Les effets des souches bactériennes de l'invention sont pleinement conservés entre l'ensemencement et les infections mycorhiziennes primaires, ce qui permet d'utiliser plusieurs méthodes d'apport.

On citera :

- l'épandage d'un inoculum bactérien avant ou après semis, ou
- le traitement bactérien des graines, en combinaison avec toute technique d'inoculation mycorhizienne, ou
- l'addition des bactéries à l'inoculum mycélien lors de sa préparation,

Les conditions pratiques d'application des souches de l'invention s'avèrent donc d'une grande simplicité, et ce d'autant plus que leur emploi supprime les besoins habituels de désinfection du sol. Leur efficacité élevée permet en outre de réduire les doses de champignon inoculé et par là le coût du procédé.

D'autres caractéristiques et avantages de l'invention sont rapportés dans les exemples qui suivent.

Afin d'illustrer l'invention, on indique ci-après le procédé d'isolement de bactéries de l'invention à partir de L.laccata et les effets observés au cours de diverses expériences. Dans ces exemples, il est fait référence aux figures 1 à 7 qui représentent respectivement :

- les figures 1 et 2, les taux de mycorhization (TM) par L.laccata après traitement avec des souches isolées,
- les figures 3 à 5, le TM par T.terrestris après traitement avec des souches isolées,
- la figure 6, les fragments de restriction d'ADN de souches de l'invention après électrophorèse sur gel d'agarose, comparés à des souches connues, et
- la figure 7, le profil polypeptidique obtenu par électrophorèse monodimensionnelle d'une souche de l'invention.

## Exemple 1 : **sélection de bactéries auxiliaires de la mycorhization**.

On rapporte dans cet exemple l'isolement de bactéries à partir de Laccaria laccata associé à de jeunes plants de Douglas et leur sélection en fonction des critères de l'invention (stimulation de la mycorhize par un champignon introduit et inhibition des champignons compétiteurs).

On utilise Laccaria laccata (Scop. ex FR) Cke, isolat S-238 de USDA (Corvallis, Oregon).

Les plants de Douglas sont obtenus à partir de graines de Douglas Pseudotsuga menziesii (Mirb).

### . Isolement

Les souches bactériennes sont isolées à partir des carpophores, et des mycorhizes stérilisées en surface, de Laccaria laccata associé à de jeunes plants de Douglas dans des expériences en pot en serre, en pépinière et en plantation.

Les sporocarpes sont nettoyés à la brosse et cassés pour les ouvrir. Des morceaux de tissus de l'intérieur du chapeau sont homogénéisés dans de l'eau stérile en utilisant un homogénéiseur Ultraturax$^R$.

Les mycorhizes sont lavées à l'eau courante, stérilisées en surface dans NaC10 1,5 %, 2 mn, rincées 20 fois dans de l'eau stérile et homogénéisées dans les mêmes conditions que les tissus de sporocarpes.

On vérifie la stérilisation de la surface en étalant l'eau du dernier rinçage sur de l'agar nutritif.

Des dilutions en séries des suspensions des carpophores et des mycorhizes sont appliquées sur un milieu TSA à 0,3 % (milieu Trypsique Soja Agar, DIFCO).

Les colonies distinctes sont isolées et remises en culture sur le même milieu.

Les isolats de mycorhizes sont désignés par Ax et ceux de carpophores par Bx, x correspondant au numéro de l'essai.

### .Criblage

On crible les isolats dont la croissance est rapide sur milieu TSA et qui stimulent la croissance de Laccaria laccata dans le test de confrontation décrit par Duponnois et Garbaye dans Can. J. Bot, 1990, 68, 2148-2152.

On indique ci-après les caractérisations morphologiques et physiologiques de divers isolats.

### .Caractéristiques morphologiques des souches bactériennes utilisées dans le criblage.

On a effectué une coloration Gram sur des colonies de 4 jours. La morphologie (MOR), la motilité (MOT) et la présence d'endospores (SPOR) ont été examinées en utilisant des suspensions de colonies de 4 jours et un microscope à contraste de phase.

Pour détecter les bactéries fluorescentes (Flu), les isolats ont été maintenus sur un milieu King's B (King et al., J. Lab. and Chim. Med. 44 : 301-307, 1954).

Les résultats obtenus sont rassemblés dans le tableau 1 ci-après :

Tableau 1

| Isolats bactériens | GRAM | MOR | MOT | SPOR | FLU |
|---|---|---|---|---|---|
| A1 | + | Grands bâtonnets | + | - | - |
| A2 | + | Grands bâtonnets | - | - | - |
| A4 | + | Grands bâtonnets | - | - | - |
| A5 | + | Chaînes de bâtonnets allongés | - | - | - |
| A10 | + | Grands bâtonnets | - | + | - |
| A14 | + | Grands bâtonnets | - | + | - |
| A15 | + | Bâtonnets minces | - | - | - |
| A8 | - | Petits bâtonnets | - | - | - |
| A9 | - | Petits bâtonnets | + | - | - |
| B4 | - | Petits bâtonnets | - | - | - |
| B5 | - | Petits bâtonnets | + | - | - |
| B6 | - | Bâtonnets courts | + | - | - |
| B7 | - | Bâtonnets courts | - | - | + |
| B8 | - | Bâtonnets courts | - | - | + |

**.Etude des caractéristiques physiologiques des isolats bactériens (Gram négatif).**

Deux tests ont été effectués sur les colonies bactériennes : l'action de la β-galactosidase (ONPG réf. 55601, bio Mérieux, France) et la présence d'oxydase cytochrome (OX : Réf 55922, bio Mérieux, France).

Les isolats Gram négatif sont ensuite examinés par le système API ZONE (API 2005), ce qui implique les déterminations suivantes :

action de la nitrate réductase (NIT) ; action de la tryptophanase (TRP) ; production de métabolites acides à partir de glucose (GLU) ; action de l'arginine dihydrolase (ADH) ; action de l'uréase (URE) ; action de la β-glucosidase (ESC) ; protéolyse de la gélatine (GEL) ; action de la β-galactosidase (ONPG) ; utilisation comme sources de carbone de glucose (GLU), d'arabinose (ARA), de mannose (MNE), de mannitol (MAN), de N-acétyl-glucosamine (NAG), de maltose (MAL), de gluconate (GNT), de caprate (CAP), d'adipate (ADI), de malate (MLT), de citrate (CIT), de phénylacétate (PAC) ; présence de cytochrome oxydase (OX).

Les résultats obtenus sont donnés dans le tableau 2 suivant :

Tableau 2

| Isolats bactériens | NIT | TRP | GLU | ADH | URE | ESC | GEL | ONPG | GLU | ARA | MNE | MAN | NAG | MAL | GNT | CAP | ADI | MLT | CIT | PAC | OX |
|---|---|---|---|---|---|---|---|---|---|---|---|---|---|---|---|---|---|---|---|---|---|
| A9 | − | − | − | − | − | + | − | − | + | + | + | + | + | + | + | − | − | + | − | − | + |
| B6 | + | − | − | − | − | + | + | + | + | + | + | + | + | + | + | − | − | + | + | − | + |
| B7 | − | − | − | + | + | − | − | − | + | + | + | + | + | − | + | + | + | + | + | + | + |
| B8 | − | − | − | + | − | − | − | − | + | + | + | + | + | − | + | + | + | + | + | + | + |
| B4 | + | − | − | − | − | + | + | + | + | + | + | + | + | + | + | − | − | + | + | − | + |
| B5 | − | − | − | − | + | + | − | + | + | + | + | + | + | + | + | + | + | + | + | − | + |

EP 0 521 758 A1

**.Etude des caractéristiques physiologiques des isolats bactériens (Gram positif).**

Les isolats à Gram positif ont été examinés par le système API 50 CHB (API 5043). Les tests suivants ont été effectués :

production de métabolites acides à partir de carbohydrates glycérol (GLY), érythrol (ERY), D-arabinose (D ARA), L-arabinose (L ARA), ribose (RIB), D-xylose (D XYL), L-xylose (L XYL), adonitol (ADO), β-méthyl-D xyloside (MDX), galactose (GAL), glucose (GLU), fructose (FRU), mannose (MAN), rhamnose (RHA), dulcitol (DUL), inositol (INO), mannitol (MAT), sorbitol (SOR), α-méthyl-D mannoside (MDM), α-méthyl -D glucoside (MDG), N acétyl glucosamine (NAG), amygdaline (AMY), arbutine (ARB), esculine (ESC), salicine (SAL), cellobiose (CEL), maltose (MAL), lactose (LAC), melibiose (MEL), sucrose (SAC), trehalose (TRE), L-sorbose (L SOR), inuline (INU), mélézitose (MLZ), raffinose (RAF), amidon (AMD), glycogène (GLG), xylitol (XLT), gentiobiose (GEN), D turanose (D TUR), D lyxose (D LYX), D tagatose (D TAG), D fucose (D FUC), L fucose (L FUC), D arabitol (D AR), L arabitol (L AR), gluconate (GNT) 2 céto gluconate (2 KG), 5 céto gluconate (5 KG).

Dans le tableau 3, on a rassemblé les résultats obtenus.

Tableau 3 :

| Tests | A1 | A2 | A4 | A5 | A10 | A14 | A15 |
|-------|----|----|----|----|-----|-----|-----|
| GLY   | +  | +  | +  | +  | +   | +   | +   |
| ERY   | –  | –  | –  | –  | –   | –   | –   |
| DARA  | –  | –  | –  | –  | –   | –   | –   |
| LARA  | +  | +  | +  | +  | +   | +   | +   |
| RIB   | +  | +  | –  | +  | +   | +   | +   |
| DXYL  | +  | +  | –  | +  | +   | +   | +   |
| LXYL  | –  | –  | –  | –  | –   | –   | –   |
| ADO   | –  | –  | –  | –  | –   | –   | –   |
| MDX   | –  | –  | –  | –  | –   | +   | +   |
| GAL   | +  | –  | +  | +  | –   | +   | +   |
| GLU   | +  | +  | +  | +  | +   | +   | +   |
| FRU   | +  | +  | +  | +  | +   | +   | +   |
| MAN   | +  | +  | +  | +  | +   | +   | +   |
| LSOR  | –  | –  | –  | –  | –   | –   | –   |
| RHA   | –  | –  | –  | –  | –   | +   | –   |
| DUL   | –  | –  | –  | –  | –   | –   | –   |
| INO   | +  | +  | –  | –  | +   | –   | –   |
| MAT   | +  | +  | +  | +  | +   | +   | +   |
| SOR   | +  | +  | –  | –  | –   | –   | –   |
| MDM   | –  | –  | –  | –  | –   | –   | –   |
| MDG   | +  | +  | +  | –  | –   | +   | +   |
| NAG   | –  | –  | –  | +  | –   | –   | +   |
| AMY   | +  | +  | +  | +  | +   | +   | +   |
| ARB   | +  | +  | +  | +  | –   | +   | +   |
| ESC   | +  | +  | +  | +  | +   | +   | +   |
| SAL   | +  | +  | +  | +  | +   | +   | +   |
| CEL   | +  | +  | +  | +  | +   | +   | +   |
| MAL   | +  | +  | +  | +  | +   | +   | +   |
| LAC   | +  | +  | +  | +  | –   | +   | +   |
| MEL   | +  | +  | +  | +  | +   | +   | +   |
| SAC   | +  | +  | +  | +  | +   | +   | +   |
| TRE   | +  | +  | +  | +  | +   | +   | +   |
| INU   | –  | –  | +  | –  | +   | +   | –   |
| MLZ   | –  | –  | –  | –  | –   | +   | –   |
| RAF   | +  | +  | +  | +  | +   | +   | +   |
| AMD   | +  | +  | +  | +  | –   | +   | +   |
| GLG   | +  | +  | +  | +  | –   | +   | +   |
| XLT   | –  | –  | –  | –  | –   | –   | –   |
| GEN   | +  | +  | +  | +  | +   | +   | +   |
| DTUR  | +  | +  | –  | +  | –   | +   | +   |
| DLYX  | –  | –  | –  | –  | –   | –   | +   |
| DTAG  | –  | –  | –  | –  | –   | +   | +   |
| DFUC  | –  | –  | –  | –  | –   | –   | –   |
| LFUC  | –  | –  | –  | –  | –   | –   | –   |
| DAR   | –  | –  | –  | –  | –   | –   | –   |
| LAR   | –  | –  | –  | –  | –   | –   | –   |
| GNT   | –  | –  | +  | –  | –   | –   | –   |
| 2KG   | –  | –  | –  | –  | –   | –   | –   |
| 5KG   | –  | –  | –  | –  | –   | –   | –   |

Les groupes dominants sont les Bacilles pour les Gram positif et les Pseudomonas pour les Gram négatif, deux d'entr'eux étant fluorescents.

On remarque que la plupart des isolats bactériens provenant de sporocarpes sont Gram négatif avec une proportion élevée de Pseudomonas.

### .Caractérisation moléculaire de B8 et A2

L'amplification génique in vitro (Polymerase Chain Reacion, PCR) suivie de l'analyse du polymorphisme de taille des fragments de restriction (RFLP) ont été appliquées à la séquence nucléotidique de l'ADN ribosomal 16S des souches B8 et A2 déposées à la C.N.C.M. respectivement sous les numéros I-1115 et I-1112 ; à titre de comparaison, deux souches de référence provenant de la D.S.M. (Deutsche Sammlung von mikroorganismen) ont également été étudiées : n° 50090 (Pseudomonas fluorescens) et n° 7 (Bacillus amyloliquefaciens).

Le protocole suivant a été mis en oeuvre :

Culture des bactéries - Les bactéries sont cultivées 48 h dans un milieu TSB liquide (Tryptic Soy Broth) à 25°C.

Amplification - Quatre µl de la culture bactérienne sont déposés dans un tube à microcentrifuger contenant 96 µl de la solution d'amplification suivante : 500 pmol de chacune des deux amorces oligonucléotidiques fD1 (AGAGTTTGATCCTGGCTCAG) et rD1 (AAGGAGGTGATCCAGCC) dans dNTP (400 µM), tampon Tris-HCl pH 8,8 (10 mM), $MgC_{P2}$ (1,5 mM), KCl (50 mM),Triton-X (0,1 %). Les amorces ont été synthétisées et fournies par Bioprobe Systems (Montreuil-sous-Bois, France).

Le tube est alors chauffé à 95°C pendant 10-20 min afin de faire éclater les cellules et de libérer l'ADN, et 2,5 unités de Taq DNA polymerase (Bioprobe Systems, Montreuil-sous-Bois, France) sont ajoutés. Une goutte d'huile minérale (Sigma Chimie) déposée à la surface de la solution évite l'évaporation. Le tube est placé dans un thermobloc GeneATAQ Controller (PharmaciaLKB) et le mélange réactionnel est soumis à 25-30 cycles d'amplification. Chacun des cycles consiste en trois phases : 2 min à 95°C (dénaturation de l'ADN), 25 s à 50°C (hybridation) et 2 min à 72°C (polymérisation). L'ensemble des 25-30 cycles est précédé d'une période de dénaturation initiale (3 min à 95°C) et suivi d'une période de polymérisation finale (10 min à 72°C).

Un témoin sans bactéries est réalisé pour vérifier l'absence de tout ADN contaminant dans les réactifs. Le degré et la spécificité de l'amplification sont évalués par électrophorèse sur gel d'agarose à 1 % (Sambrook et al., 1989) Molecular cloning : a laboratory manual. Cold Spring Harbor, New York).

Analyse de la taille des fragments de restriction - De 1 à 2 µg d'ADN amplifié sont digérés pendant 12-14 h par 5 unités de l'une ou l'autre des enzymes de restriction suivantes : AluI, HinfI, MboI ou RsaI selon les instructions du fournisseur (Pharmacie Fine Chemicals, Biolabs). les fragments de restriction sont soumis à une électrophorèse sur gel d'agarose à 2 % (Sambrook et al., référence précitée) ; les bandes correspondantes sont colorées au bromure d'éthidium et photographiées sous lumière ultra-violette. Comme standards de taille des fragments révélés, on fait migrer sur le même gel de l'ADN ϕX174 (Pharmacia Fine Chemicals, Biolabs) préalablement soumis à l'enzyme de restriction HaeIII.

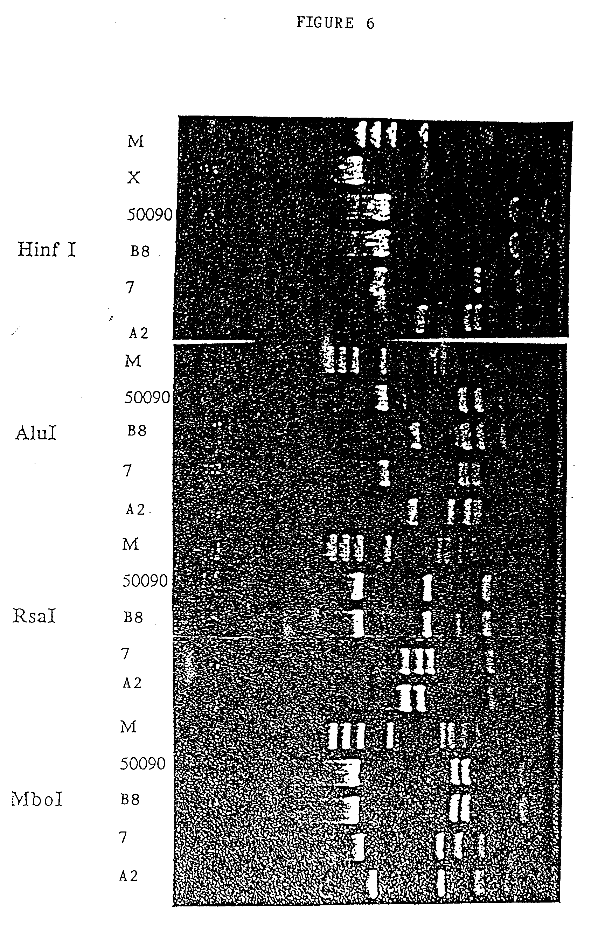

La photographie rapportée sur la figure 6 montre les bandes obtenues, caractéristiques de chacune des quatre souches bactériennes étudiées, sur deux gels (gel 1: avec les enzymes de restriction MboI, RsaI et AluI ; gel 2 : avec l'enzyme de restriction HinfI). M : standard (bande 1 : 1358 paires de bases ; bande 4 : 603 paires de bases ; bande 8 : 194 paires de bases). X : ADN bactérien 16S amplifié mais non coupé.

Le tableau 4 suivant donne les résultats obtenus en ce qui concerne les tailles approximatives en paires de bases (pb) des fragments de B8, exprimées :

## TABLEAU 4

|                                    | B8   | DSM n° 50090 |
|------------------------------------|------|--------------|
| séquence variable V2               | 120  | 120          |
| séquence variable V6               | 100  | 100          |
| V2+séquence intermédiaire+V6       | 1000 | 1000         |
| fragments de restriction           | 700  | 700          |
|                                    | 250  | 250          |
|                                    | 100  | 100          |

Les résultats obtenus confirment l'appartenance de la souche B8 à l'espèce <u>Pseudomonas fluorescens</u>.

L'ADN simple brin de la région V2+séquence intermédiaire+V6 de la souche B8, amplifiée par PCR comme précédemment, a ensuite été séquencée par l'ADN polymerase du phage T7 (selon la méthode de Sanger dans Proc. Nat. Acad. Sci. USA, 74, 5463-5467, 1977). La séquence suivante, caractéristique de la souche, a été mise en évidence :

5'-ATGTAAGGCCATGGTAAGTTCTTCGACGATTT-3'

La souche B8 a également fait l'objet de la caractérisation de son profil polypeptidique par électrophorèse monodimensionnelle en conditions dénaturantes sur gel discontinu de polyacrylamide d'après la technique de Laemmli dans Nature, 227, 680-685, 1970. La bactérie a été cultivée 48 h, homogénéisée, et les protéines précipitées à l'acétone à -20°C. Après centrifugation, le culot protéique a été solubilisé dans le tampon de migration de Laemmli. La concentration en polyacrylamide du gel de concentration était de 5 % et celle du gel de séparation de 15 %, et l'intensité du courant de 15 puis 30 mA. Après révélation des polypeptides par coloration argentique, le gel a été séché et les densités optiques mesurées au spectrophotomètre à 540 nm. Les masses molaires correspondant aux différents pics ont été déterminées en faisant migrer des protéines de référence sur le même gel. La figure 7 donne le profil polypeptidique obtenu, avec les mesures d'absorbance en ordonnées et de position des pics sur le gel en mm, en abcisse. L'indication des poids moléculaires est rapportée dans la partie haute de la figure.

<u>Exemple 2</u>: **Caractérisation des souches selon leurs effets agonistes et antagonistes définis plus haut.**

**a) Expériences en serre :**

**- Etude de l'effet des isolats bactériens sur l'infection mycorhizienne de Douglas par <u>L.laccata</u>.**

**. conditions des expériences.**

Les trois composants du système (bactérie, champignon et plante) sont mis en confrontation dans des godets en polyéthylène de 95 ml remplis avec un mélange non désinfecté de vermiculite et de tourbe (1/1. vol/vol) et d'inoculum fongique (1/10 ; vol/vol). A l'aide d'une seringue, on injecte 5 ml dans chaque récipient d'une suspension bactérienne concentrée (plus de $10^8$ cellules/ml) dans 0,1 M $MgSO_4$, $7H_2O$. Dans un traitement témoin, on inocule le champignon et la solution tampon, sans bactérie. On ensemence 3 graines par godet.

Lorsqu'elles atteignent le stade cotylédon, on éclaircit les plantules afin d'en avoir une par godet. Chaque traitement est représenté par un plateau contenant 40 cellules.

A partir de 5 semaines, on applique en excès deux fois par semaine, en ajoutant également de l'eau chaque jour, une solution nutritive (14,8 mg/l d'azote à partir de nitrate et 2 mg/l de phosphore) dont on a vérifié auparavant l'effet favorable sur le développement de la mycorhize.

On déplace chaque mois les plateaux sur les tablettes de la serre pour compenser les gradients microclimatiques.

On rapporte les résultats obtenus dans deux expériences réalisées dans des conditions climatiques différentes, la première en été (à une température de 15 à 28°C), la deuxième en hiver (10 à 20°C).

La photopériode (16 heures) était la même dans les deux expériences (lumière du jour complémentée par de la lumière artificielle).

On a mesuré le taux de mycorhization en déterminant le nombre de racines courtes mycorhizées par rapport au nombre total de racines courtes, et transformé par arc sin (racine carrée). Tous les résultats sont traités statistiquement, les différences sont considérées comme significatives au risque de 5 %.

La valeur moyenne du taux de mycorhization de chaque traitement a été comparée à celle du témoin.

**.Expérience d'été.**

On prélève 10 semis par traitement, plusieurs semaines après l'ensemencement.

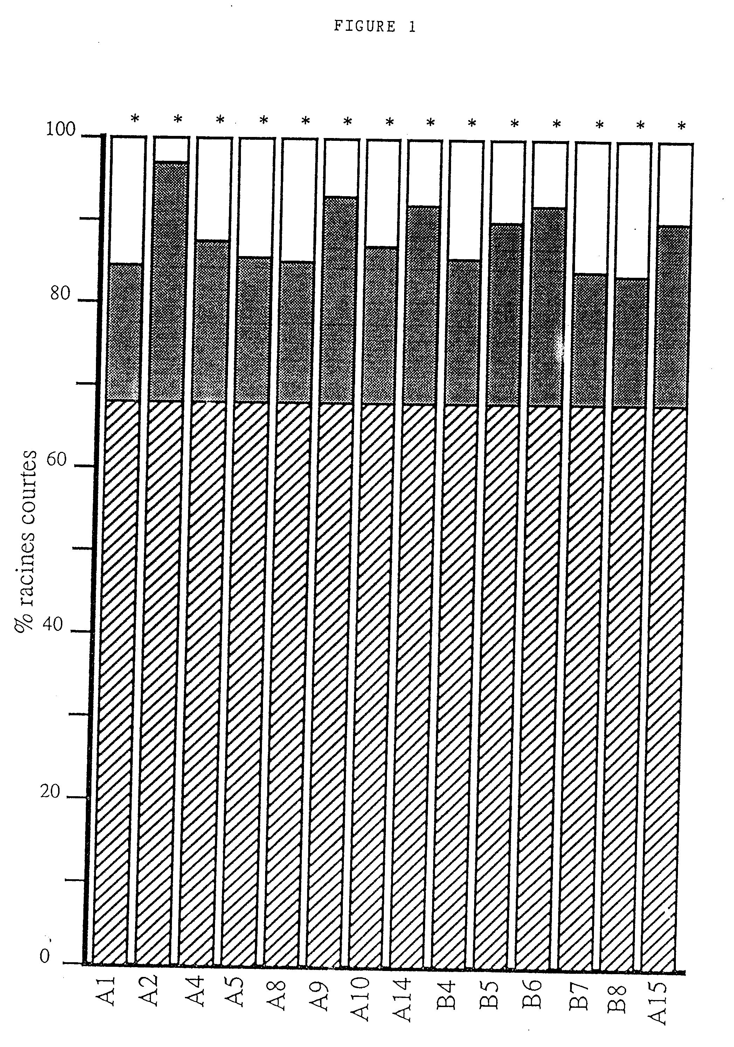

Sur la figure 1, on rapporte les résultats obtenus après 16 semaines, avec les isolats bactériens.

$A_1$ à $A_9$ (prélevés de mycorhizes) et $B_1$ à $B_5$ (prélevés de sporocarpes).

Les légendes de cette figure sont les suivantes:
- la partie vide correspond aux racines courtes non mycorhizées.
- la partie avec les hachures, à l'infection par L.laccata chez le témoin,
- la partie en pointillés, à l'infection ectomycorhizienne due à l'inoculation bactérienne.

A la 16ème semaine, le pourcentage de racines courtes dans cette expérience est de 67 % chez le témoin.

En revanche, avec les isolats testés, ce pourcentage varie de 83 % à 97 %, mettant ainsi en évidence l'effet fortement favorable des bactéries sélectionnées.

**.Expérience d'hiver.**

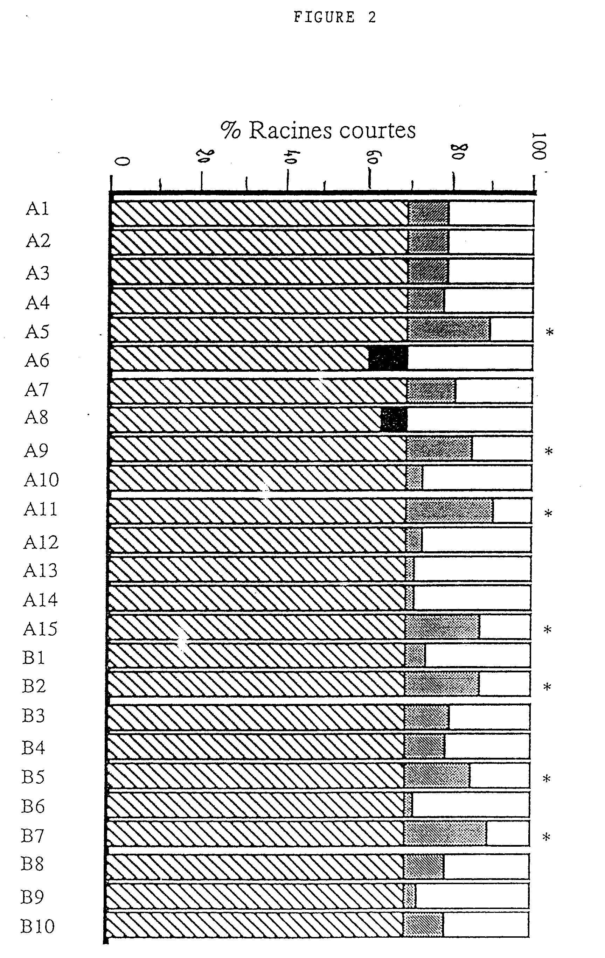

Les résultats obtenus après 18 semaines de traitement sont rapportés sur la figure 2 et concernent les isolats bactériens A1 à A15 et B1 à B10. Les légendes de cette figure sont identiques à celles de la figure 1.

L'examen de ces résultats montre que le taux de mycorhize qui est de 70 % environ chez le témoin dépasse 80 % pour A5, A9, A11, A15, B2, B5, BBc3, atteignant même 93 % avec A11.

On remarque que certaines des souches bactériennes exercent un effet auxiliaire de la mycorhization dans les deux conditions de température (été et hiver), ce qui permet d'envisager un large domaine d'application du point de vue micro-climatique.

**-Etude de l'effet sur T.terrestris**

On a testé dans des conditions d'hiver, des isolats bactériens criblés comme indiqué plus haut, avec ou sans inoculation de L.laccata pour déterminer leur effet sur l'infection des semences par T.terrestris.

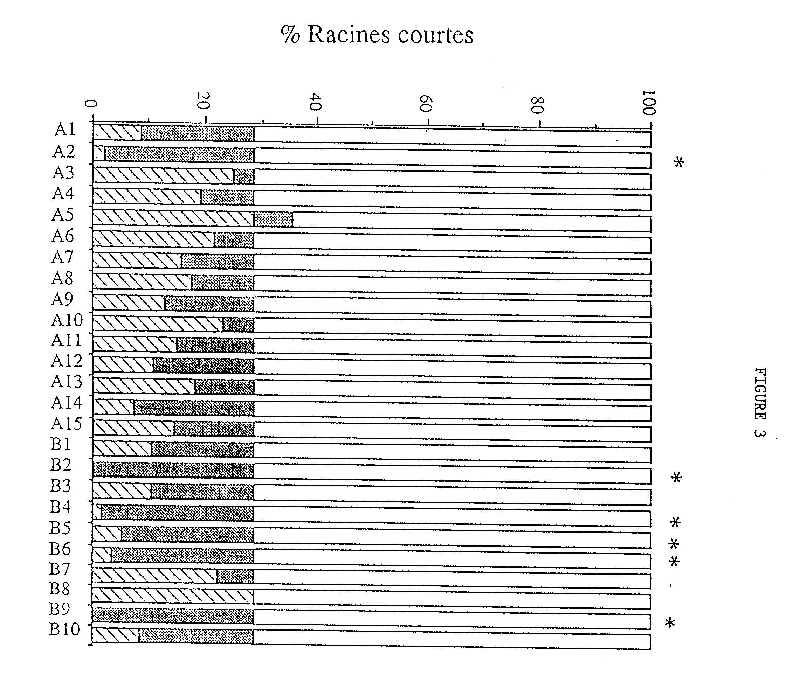

Les résultats obtenus à la 18ème semaine sont rapportés sur la figure 3, Sur cette figure, la partie hachurée correspond au témoin sans addition de bactéries de l'invention, et la partie en noir, au déficit de mycorhizes résultant de l'effet des bactéries.

Les isolats testés sont ceux de l'exemple précédent, à savoir A1 à A15 et B1 à B10.

On constate dans chaque cas une forte diminution du taux de mycorhization par T.terrestris, l'inhibition étant totale avec B2 et B9.

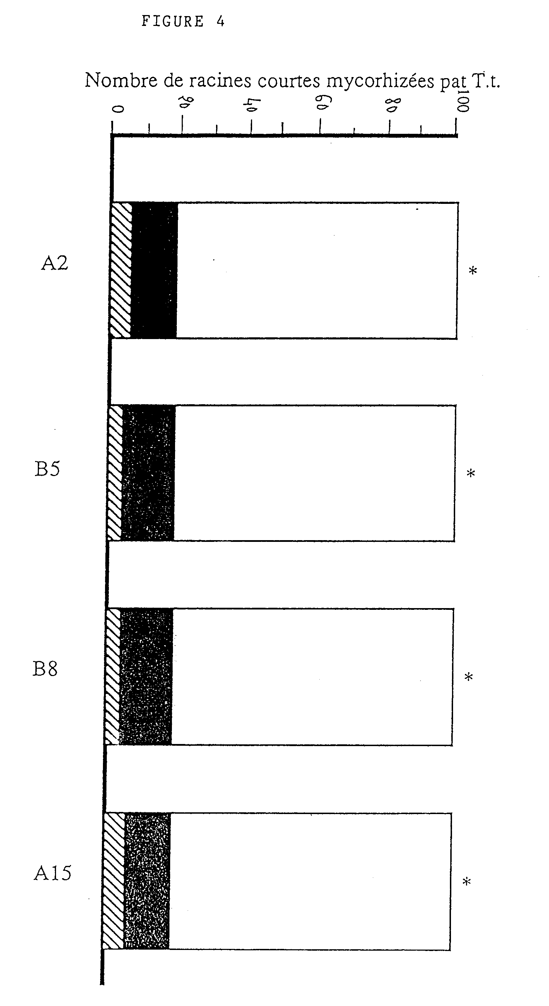

- On rapporte sur la figure 4 les résultats d'expériences en serre, sur un mélange tourbe/vermiculite avec A2, B5, B8 et A15.

Les légendes sont les mêmes que sur la figure 3. On observe également une forte diminution de la mycorhization par T.terrestris sous l'effet de l'application de bactéries selon l'invention.

**b) Expériences sur sol de pépinière :**

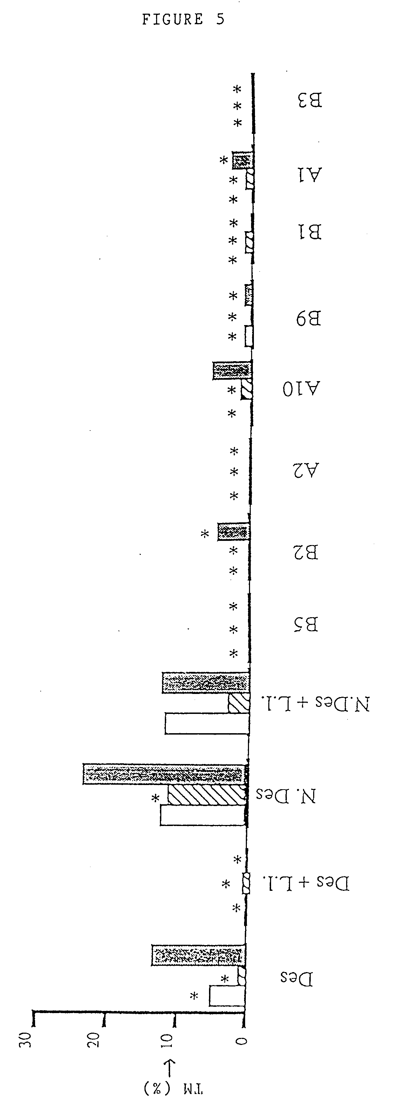

Sur la figure 5, on rapporte les résultats d'expériences en serre sur sol de pépinière.

Cette figure indique que le taux de mycorhization (en %) par T.terrestris dans 5 traitements effectués respectivement sur sol :
- désinfecté (Des),

- désinfecté et traité par L.laccata (Des+ L.l),
- non désinfecté (N Des),
- non désinfecté et traité par L.laccata (N Des + L.l),
- traité par un isolat tel qu'obtenu à l'issue du criblage, à savoir B1, B2, B3, B5, A1, A2 et A10.

Les résultats sont donnés après 12 semaines de traitement (partie vide), de 16 semaines (partie hachurée) et de 21 semaines (partie en pointillés).

Les résultats confirment l'efficacité de l'inhibition du développement de T.terrestris par les bactéries de l'invention.

**c) expériences in vitro sur L.laccata :**

On rapporte les résultats concernant les isolats bactériens B5 et B8, A2, A4, A8, B6, A15, choisis parmi ceux donnant lieu à la stimulation la plus élevée de l'infection mycorhizienne dans l'expérience en serre d'été.

Les trois composants du système (bactérie, champignon, plante) sont mis en confrontation dans des conditions aseptiques dans des tubes à essais en verre (3 x 15 cm) remplis avec un mélange vermiculite-tourbe (1/1 ; vol/vol) traité à l'autoclave (120°C, 20 min), humidifié avec la solution nutritive utilisée dans la serre et mélangée avec l'inoculum fongique 1:10 (vol:vol).

On injecte dans chaque tube, à l'aide d'une seringue, 1 ml d'une suspension bactérienne concentrée (plus de $10^8$ cellules/ml) dans $MgSO_4$, 7 $H_2O$ 0,1 M stérile. Dans un traitement témoin, on n'utilise que la solution tampon. Les tubes, sont recouverts d'une feuille d'aluminium et autoclavés. On introduit dans un trou de la feuille la petite racine d'une graine germée aseptiquement. Après avoir scellé, au niveau du collet avec un joint étanche, les tubes sont placés en chambre de croissance.

Les racines sont alors maintenues dans des conditions axéniques, tandis que la partie aérienne de la plante se développe librement à l'extérieur du tube. On laisse pousser les plantes 4 semaines dans une pièce au climat contrôlé (23°C le jour, 17°C la nuit, 16 heures de photopériode avec 400 $\mu E.m^{-2}S^{-1}$, 80 % d'humidité). La détermination du taux de mycorhization, effectué comme dans les expériences précédentes, montre dans chaque cas une forte augmentation par rapport au témoin.

Ces résultats obtenus axéniquement prouvent sans ambiguïté que l'effet de stimulation de l'infection mycorhizienne est propre à la souche introduite.

**Exemple 3: effets de bactéries de l'invention sur la mycorhization du Douglas par différents champignons ectomycorhiziens en pépinière.**

Différents champignons ectomycorhiziens ont été utilisés dans cette expérience : Laccaria laccata S238 (témoin positif), Laccaria bicolor D-101 (témoin positif), Hebeloma cylindrosporum D-15, Laccaria proxima 415 et Paxillus involutus QBC.

L'inoculum fongique a été préparé en utilisant la technique indiquée plus haut dans l'exemple 1.

Des isolats bactériens B5 et B8 ont été cultivés pendant 8 jours sous agitation dans des erlens de 300 ml contenant 100 ml de milieu TSB 0,3 % dans une chambre de culture (25°C, obscurité). Ces suspensions ont ensuite été centrifugées (2400g, 10 min), le surnageant a été éliminé et les cellules bactériennes ont été remises en suspension dans 100 ml de sulfate de magnésium 0,1 M. Cette suspension bactérienne a été diluée dans 7,5 litres d'eau osmosée. La suspension finale contenait environ $10^{12}$ cellules bactériennes. Pour le traitement témoin (sans bactérie), 100 ml de $MgSO_4$ 0,1M ont été dilués dans 7,5 litres d'eau.

L'inoculation fongique a été réalisée à raison de 2 litres d'inoculum par m². Un traitement non inoculé a été effectué.

Les bactéries diluées dans de l'eau ont été inoculées à l'aide d'un arrosoir à raison de 2,5 litres par placeau ($10^{12}$ cfu.m$^{-2}$).

Ces deux applications ayant été réalisées, les 2 inoculums ont été mélangés au sol jusqu'à une profondeur de 10 cm environ.

Après 4 mois de culture, 10 semis ont été prélevés au hasard dans chaque placeau. Le taux de mycorhization (nombre de racines courtes mycorhizées/nombre de racines courtes totales) a été déterminé sur un échantillon de 100 racines courtes.

Les résultats obtenus montrent l'effet de stimulation de la mycorhization par L-laccata et L-bicolor exercé par B5 et B8 en pépinière. Ces résultats sont confirmés en conditions axémiques.

Exemple 4 : effets de bactéries de l'invention sur des mycorhizes de L.laccata avec le chêne pédonculé (inoculation séquentielle).

On rapporte ci-après les expériences réalisées dans deux pépinières situées dans des lieux différents avec les souches bactériennes B2 et B8 de l'exemple 1.

On utilise des semis de chênes pédonculé (Quercus robur L) obtenus à partir de glands ramassés à l'automne, trempés dans de l'eau à 41°C pendant deux heures (pour empêcher la nécrose des cotydélons provoquée par Ciboria batschiana), poudrés avec un gramme par kilo d'iprodione (contre Penicillium spp. et autres moisissures de conservation) et maintenus à 4°C.

On mélange L.laccata dans des lits de tourbe de la pépinière à la dose de 1,1 l d'inoculum/m², puis on sème les glands.

La souche de L.laccata est celle utilisée dans les autres exemples portant sur le Douglas.

On fait pousser les semis sous un grand tunnel de polyéthylène (6 x 42 m) dans une couche de tourbe de 20 cm d'épaisseur arrosée avec un système de brouillard. La même tourbe dans le même tunnel a été préalablement utilisée pendant 7 ans pour la croissance de chênes ou de hêtres en alternance afin d'éviter l'apparition de maladies racinaires telles Pythium ultimum.

On inocule les bactéries en utilisant une suspension dans MgSO$_4$ 0,1 M.

La croissance des bactéries est effectuée pendant une semaine dans 500 ml d'un milieu TSB (Trysic Soy Broth) sur une table d'agitation à 25°C.

Les cultures qui sont alors en phase stationnaire sont centrifugées dans des tubes de 300 ml à 2400 g pendant 15 minutes et le culot est remis en suspension dans 600 ml de MgSO$_4$ 0,1 M.

Dans la pépinière, on dilue 200 ml de cette suspension dans 10 litres d'eau, et on l'applique sur le sol, le traitement témoin étant effectué avec la même quantité de sulfate de magnésium.

On observe une augmentation des taux de mycorhization de 30 à 53 %. Ces résultats mettent en évidence que la stimulation, par les bactéries de l'invention, de la formation de mycorhizes par les champignons s'exerce dans une large gamme de conditions. Ces effets sont observés aussi bien en synthèse axénique in vitro, qu'en serre sur des semis dans des conteneurs, ou en pépinières. Ces résultats montrent en outre que les effets des bactéries de l'invention s'exercent sur des plantes hôtes appartenant à des groupes taxonomiques très différents.

**Exemple 5: effet de l'inoculation de Douglas avec, conjointement, L.laccata et des bactéries de l'invention (inoculation mixte).**

On rapporte ci-après, les expériences effectuées dans des pépinières forestières.

Bactéries : on utilise cinq souches bactériennes obtenues selon l'exemple 1, à savoir :
- A2 (Bacillus subtilis)
- B6 (Pseudomonas sp.)
- B8 (Pseudomonas fluorescens)
- B5 (Pseudomonas sp)
- A15 (Bacillus sp)

Ces souches sont cultivées pendant 8 jours à 25°C dans des flacons en verre contenant du milieu liquide TSB à la concentration de 3 g/l. Les suspensions bactériennes concentrées (plus de 10$^{10}$ cellules par ml) sont centrifugées (2400 g, 10 min), le surnageant est éliminé et le culot de centrifugation est remis en suspension dans 200 ml de MgSO$_4$ 0,1 M ou dans 500 ml d'eau stérile (dans le cas d'un inoculum d'alginate).

Les concentrations sont déterminées par mesure de l'absorbance ($\alpha = 520$ nm) des suspensions, les corrélations entre l'absorbance et la concentration en cellules vivantes ayant été établies au préalable.

Préparation de l'inoculum d'alginate avec le mycélium et des bactéries.

Le mycélium de Laccaria laccata mélangé et les bactéries sont remis en suspension dans une solution d'alginate de sodium (10 g/litre) contenant de la tourbe broyée (50 g/litre). Des petites billes de 4 mm de mycélium et de bactéries sont obtenues en faisant goutter la suspension dans 100 g par litre de chlorure de calcium.

Expériences en pépinières dans deux lieux différents :

Expérience A

Le sol de la pépinière est désinfecté avec du bromure de méthyle à froid.

Laccaria laccata est inoculé à raison de 2 litres d'inoculum de tourbe-vermiculite par m². On effectue cinq traitements bactériens avec chacune des bactéries ci-dessus, inoculées à trois doses différentes : $10^5$, $10^7$ et $10^9$ cfu par m² pour B6 et $10^6$, $10^8$ et $10^{10}$ cfu par m² pour les 4 autres.

On applique cinq litres (200 ml de suspension bactérienne dans MgSO4 0,1 M et 4,5 litres d'eau) d'inoculum bactérien avec un arrosoir sur la surface de chacune des parcelles traitées, après mélange de l'inoculum fongique solide dans les 10 cm supérieurs du sol. La solution pour le traitement témoin renferme 200 ml de $MgSO_4$ 0,1 M et 4,5 litres d'eau. Aucun arrosage n'est effectué durant la saison de croissance qui était particulièrement sèche.

Le taux de mycorhization a été évalué comme indiqué ci-dessus. On a également déterminé le poids sec des pousses et des racines.

Après 4 mois, on observe un taux de mycorhization dans le témoin de 60 %. On constate que les traitements bactériens avec A2, A15, et B8 augmentent significativement l'établissement de la symbiose ectomycorhizienne. Les taux de mycorhization avec ces bactéries sont en effet respectivement de 88 %, 85 % et 83 %.

On note dans chaque cas une augmentation de la biomasse.

Expérience B

On a rempli une planche vide d'une pépinière expérimentale avec du sol ramassé sur une profondeur de 0 à 20 cm dans la pépinière de l'expérience précédente.

Le sol a été désinfecté comme décrit ci-dessus. La planche a été divisée en parcelles, séparées les unes des autres par des zones non inoculées et non ensemencées. Les parcelles ont été inoculées avec L.laccata (1 litre de billes d'alginate par m², contenant le champignon et la bactérie).

Un traitement d'inoculation a été effectué :

2 grammes (poids sec) par m² et $10^{12}$ bactérie cfu par m². Trois traitements bactériens ont été réalisés (A15, A2 et B5). Les billes d'alginate dans les témoins étaient sans bactérie. La culture a été maintenue bien arrosée durant toute la saison de croissance.

Après 3 et 5 mois de culture, les taux de mycorhization dans les témoins sont respectivement de 26 % et 83 %.

Après 3 mois, les trois isolats bactériens augmentent significativement l'infection mycorhizienne, les taux de mycorhization allant de 68 % à 82 %. Au cinquième mois, on constate un effet similaire avec des taux de mycorhization allant de 97 % 99 %.

La biomasse apparaît également augmentée lorsqu'on applique les bactéries de l'invention encapsulées avec L.laccata.

Après 3 mois de culture, la biomasse moyenne au-dessus du sol est significativement plus élevée avec le traitement avec B5 (108 mg par semis) que dans le témoin (62 mg par semis).

Après 5 mois de culture, la matière sèche des pousses et des racines dans le témoin est de 320 mg et 115 mg par semis. Les trois traitements bactériens augmentent significativement la croissance des semis : poids des pousses de 493 à 546 mg par semis et poids des racines, de 174 à 201 mg par semis.

On observe dans les deux pépinières, une croissance des parties aériennes des plantes, corrélée positivement à la vitesse d'infection mycorhizienne de leur système racinaire.

Des expériences réalisées en utilisant des billes d'alginate renfermant les bactéries et le champignon, en apportant au sol différentes quantités d'inoculum, montrent que les taux de mycorhization obtenus sont pratiquement identiques quelle que soit la quantité d'inoculum apportée. En mettant à profit l'action des bactéries de l'invention, on réduira donc avantageusement la quantité du champignon inoculé.

D'une manière générale, les résultats obtenus avec les souches bactériennes de l'invention montrent qu'elles agissent de manière spécifique sur le symbiote fongique et stimulent par cette action la mycorhization de la plante hôte.

Ce phénomène de spécificité a été retrouvé au niveau de l'effet des bactéries par voie gazeuse et aqueuse sur la croissance saprophytique de divers symbiotes fongiques. Des essais réalisés ont ainsi montré que l'acide citrique produit par une bactérie intervient dans la stimulation de la croissance d'un champignon ectomycorhizien donné.

Ces bactéries sont particulièrement utiles dans les techniques de mycorhization contrôlée en pépinière forestière sur sols désinfectés ainsi que de manière avantageuse sur sols non désinfectés, ce qui permet d'éviter l'emploi de désinfectants comme le bromure de méthyle.

## Revendications

**1/** Souches bactériennes, caractérisées en ce qu'elles sont capables de stimuler spécifiquement la formation de mycorhizes par des champignons mycorhiziens inoculés, ces souches exerçant un effet antagoniste vis-à-vis de champignons contaminants compétiteurs du champignon inoculé.

**2/** Souches selon la revendication 1, caractérisées en ce qu'elles sont capables d'augmenter significativement le taux de mycorhization par le champignon inoculé d'au moins 10 % environ par rapport au système sans apport de bactéries et d'inhiber le développement de tout champignon compétiteur de telle sorte que le taux de racines mycorhizées formées par ce champignon soit significativement réduit à une valeur inférieure à 10 % environ.

**3/** Souches selon la revendication 1 ou 2, caractérisées en ce qu'il s'agit de bactéries à Gram positif du genre Bacillus, telles que B. subtilis.

**4/** Souches selon la revendication 1 ou 2, caractérisées en ce qu'il s'agit de bactéries à Gram négatif du genre Pseudomonas, telles que P. fluorescens.

**5/** Souches bactériennes déposées à la C.N.C.M. le 11 juin 1991, sous les n° I-1112 (souche de Bacillus subtilis), I-1113 (souche de Bacillus sp.), I-1114 (souche de Pseudomonas sp.) et I-1115 (souche de Pseudomonas fluorescens).

**6/** Souches selon l'une quelconque des revendications précédentes caractérisées en ce qu'elles proviennent de la rhizosphère des arbres forestiers.

**7/** Souches selon l'une quelconque des revendications précédentes, caractérisées en ce qu'elles proviennent des manteaux des mycorhizes et des carpophores de champignons ectomycorhiziens.

**8/** Souches selon l'une quelconque des revendications 6 ou 7, caractérisées en ce qu'elles proviennent de la rhizosphère d'essences forestières à ectomycorhizes de résineux comme le Douglas, les épicéas, les pins, les mélèzes, les tsugas ou d'espèces feuillues comme les chênes, les hêtres ou les eucalyptus.

**9/** Souches selon l'une quelconque des revendications précédentes, caractérisées en ce qu'elles constituent des auxiliaires de mycorhization de champignons ectomycorhiziens d'essences forestières sélectionnées pour leur efficacité symbiotique et que l'on souhaite introduire par inoculation, tels que L.laccata, Hebeloma sp, Paxillus involutus, Rhizopogon sp, Suillus sp, Sclerodorma sp.

**10/** Souches selon l'une quelconque des revendications précédentes, caractérisées en ce qu'elles exercent un effet antagoniste vis-à-vis de T.terrestris ou de souches indésirables de Rhizopogon sp, Suillus sp et Sclerodorma sp.

**11/** Souches bactériennes, caractérisées en ce qu'elles sont telles qu'obtenues par :
- mise en suspension de carpophores et de manteaux de mycorhizes prélevés sur Laccaria laccata en symbiose avec le Douglas,
- culture en milieu approprié tel que TSA, selon les techniques habituelles,
- addition à un inoculum de L.laccata,
- ensemencement avec les graines de Douglas,
- sélection in vitro, en serre ou en pépinière, des isolats bactériens capables de donner lieu à un taux de mycorhization d'au moins 80 % par le champignon introduit et d'inhiber pratiquement totalement la formation de mycorhizes par des champignons mycorhiziens compétiteurs, tels que T.terrestris.

**12/** Procédé d'obtention de souches bactériennes selon la revendication 1, caractérisé en ce qu'il comprend
- la mise en suspension de carpophores et de mycorhizes prélevées sur des champignons mycorhiziens associés à des plantes hôtes,
- la dilution en série de ces suspensions et leur application sur un milieu approprié pour leur croissance,
- la sélection des isolats capables de donner lieu à un taux de mycorhization, sur un champignon mycorhizien inoculé à une plante hôte, significativement accru d'au moins 10 % environ par rapport à un système sans apport de bactéries, et d'inhiber pratiquement totalement l'établissement de champignons contaminants, compétiteurs vis-à-vis du champignon de l'inoculum.

**13/** Procédé de mycorhization contrôlée de plantes, caractérisé en qu'il comprend l'utilisation d'une quantité efficace d'une souche selon l'une quelconque des revendications 1 à 11.

**14/** Procédé selon la revendication 13, caractérisé en ce qu'il est mis en oeuvre sur des espèces forestières à ectomycorhizes comme les résineux, tels que le Douglas, les épicéas, les pins, les mélèzes et les tsugas, ou des espèces feuillues comme les chênes, les hêtres et les eucalyptus.

**15/** Compositions utilisables pour la mycorhization contrôlée de plantes, en ce qu'elle comprend une souche bactérienne selon l'une quelconque des revendications 1 à 11, associée à un véhicule inerte, se présentant notamment sous forme d'une suspension renfermant avantageusement de $10^7$ à $10^{12}$ bactéries/l, de préférence de $10^8$ à $10^{10}$, et contenant des adjuvants nécessaires à la conservation tels que $MgSO_4$, du polyéthylène glycol ou du glycérol, ou sous forme d'un inoculum mixte avec le champignon, la souche étant présente à raison de $10^6$ à $10^9$ bactéries par g de poids sec de mycélium.

**16/** Composition selon la revendication 15, caractérisée en ce qu'elle renferme L.laccata associé à des bactéries selon l'une quelconque des revendications 1 à 11, en particulier aux bactéries selon la revendication 5.

**17/** Méthode d'application des souches bactériennes selon l'une quelconque des revendications 1 à 11, caractérisée en ce qu'elle comprend :
- l'épandage d'un inoculum bactérien avant ou après le semis, ou
- le traitement bactérien des graines, en combinaison avec toute technique d'inoculation mycorhizienne connue à ce jour, ou
- l'addition des bactéries à l'inoculum mycélien lors de sa préparation.

FIGURE 1

FIGURE 2

FIGURE 3

FIGURE 4

Nombre de racines courtes mycorhizées pat T.t.

FIGURE 5

FIGURE 6

FIGURE 7

Office européen
des brevets

**RAPPORT DE RECHERCHE EUROPEENNE**

Numero de la demande

EP 92 40 1826
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | SYMBIOSIS<br>vol. 9, 1990,<br>pages 267 - 273<br>GARBAYE, J., ET AL. 'The bacteria associated with Laccaria Laccata ectomycorrhizas or sporocarps: Effect on symbiosis establishment on douglas fir'<br>* le document en entier *<br>--- | 1-11,<br>15-17 | C12N1/20<br>C12Q1/24<br>A01N63/00 |
| P,X | ANN SCI FOR<br>vol. 48, no. 3, 1991,<br>pages 239 - 251<br>DUPPONOIS R., ET AL. 'Mycorrhization helper bacteria associated with Douglas fir-Laccaria laccata symbiosis:effects in aseptic and in glasshouse conditions'<br>* le document en entier *<br>--- | 1-17 | |
| P,X | BIOLOGICAL ABSTRACTS vol. 93<br>, 1992, Philadelphia, PA, US;<br>abstract no. 40107,<br>DUPONNOIS, R. ET AL. 'Effect of dual inoculation of Douglas fir with the ectomycorrhizal fungus Laccaria laccata and mycorrhization helper bacteria (MHB) in two bare-root forest nurseries'<br>* abrégé *<br>& PLANT SOIL<br>vol. 138, no. 2, 1991,<br>pages 169 - 176<br>--- | 17 | |

**DOMAINES TECHNIQUES RECHERCHES (Int. Cl.5)**

A01N
C12N
C12Q

-/--

Le présent rapport a été établi pour toutes les revendications

| Lieu de la recherche | Date d'achèvement de la recherche | Examinateur |
|---|---|---|
| LA HAYE | 05 OCTOBRE 1992 | MADDOX A.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen
des brevets

# RAPPORT DE RECHERCHE EUROPEENNE

Numero de la demande

EP   92 40 1826
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| P,X | BIOLOGICAL ABSTRACTS vol. 94, 1992, Philadelphia, PA, US; abstract no. 4281, GARBAYE, J., ET AL. 'Effects of substrate sterilization, fungicide treatment, and mycorrhization helper bacteria on ectomycorrhizal formation on penunculate oak (Quercus robur) inoculated with Laccaria laccata in two peat bare-root nurseries' * abrégé * & BIOL FERTIL SOILS vol. 13, no. 1, 1992, pages 55 - 57 | 17 | |
| A | EXPERIENTIA vol. 47, no. 4, Avril 1991, pages 370 - 375 GARBAYE J. 'Biological interactions in the mycorrhizosphere' * le document en entier * | 1-17 | |
| | | | DOMAINES TECHNIQUES RECHERCHES (Int. Cl.5) |

Le présent rapport a été établi pour toutes les revendications

| Lieu de la recherche | Date d'achèvement de la recherche | Examinateur |
|---|---|---|
| LA HAYE | 05 OCTOBRE 1992 | MADDOX A.D. |